# EUROPEAN PATENT SPECIFICATION

(11) **EP 1 749 812 B1**
(45) Date of publication and mention of the grant of the patent: **26.03.2014**
(21) Application number: 04792053.3
(22) Date of filing: 05.10.2004
(51) Int. Cl.: C07C 51/50, C07C 57/075, C07C 67/62, C07C 69/54

(54) **PROCESS FOR PRODUCING EASILY POLYMERIZABLE COMPOUND WITH A POLYMERIZATION INHIBITOR**
VERFAHREN ZUR HERSTELLUNG EINER LEICHT POLYMERISIERBAREN VERBINDUNG MIT EINEM POLYMERISATIONSINHIBITOR
PROCESSUS DE FABRICATION DE COMPOSÉ FACILEMENT POLYMÉRISABLE GRÂCE À UN INHIBITEUR DE POLYMÉRISATION

(30) Priority: 26.05.2004 JP 2004155782
(43) Date of publication of application: 07.02.2007
(73) Proprietor: Mitsubishi Chemical Corporation, Chiyoda-ku Tokyo 100-8251 (JP)
(72) Inventor: TAKASAKI, Kenji, MITSUBISHI CHEMICAL CORPORATION, Yokkaichi-shi, Mie 5108530 (JP); YADA, Shuhei, MITSUBISHI CHEMICAL CORPORATION, Minato-ku, Tokyo 108-0014 (JP); KATOU, Tomokazu, MITSUBISHI CHEMICAL CORPORATION, Yokkaichi-shi, Mie 5108530 (JP); NUMATA, Shigeaki, KAWASAKI KASEI CHEMICALS LTD., Kawasaki-shi, Kanagawa 2100826 (JP)
(74) Representative: HOFFMANN EITLE
(86) International application number: PCT/JP2004/014642
(87) International publication number: WO 2005/115958

(56) References cited:
- EP-A- 0 297 583
- US-A- 3 563 742
- NOBASHI K. ET AL: 'Inhibition of vinyl polymerization with extractives from Keyaki wood' MOKUZAI GAKKAISHI vol. 23, no. 12, 1977, pages 670 - 675, XP002996976
- YOKOTA T. ET AL: 'The inhibitory effects of extractives prepared from Keyaki heartwood on vinyl polymerizations' MOKUZAI GAKKAISHI vol. 22, no. 11, 1976, pages 632 - 637, XP002996977

## Description

### TECHNICAL FIELD

The present invention relates to the use of a compound of formula (II) as a polymerization inhibitor applicable to an easily polymerizable compound, having a polymerizable double bond in a molecule selected from acrylic acid, methacrylic acid (hereinafter, both of them are collectively referred to as "(meth)acrylic acids"), and (meth) acrylate. In addition, the present invention relates to a method of producing an easily polymerizable compound using the polymerization inhibitor.

### BACKGROUND ART

There have been proposed various compounds as polymerization inhibitors to be added to (meth) acrylic acid and esters thereof. Examples of the various compounds include: a phenol-based polymerization inhibitor such as hydroquinone or cresol (see, for example, JP-A 54-14904 and JP-A 5-320095); an amine-based polymerization inhibitor such as o-phenylenediamine, diallyl-p-phenylenediamine, or a derivative of p-phenylenediamine (see, for example, JP-A 52-23017, JP-A 52-62219, JP-A54-14903, JP-A 1-230543, and JP-A 2-248402) ; a nitroso compound (see, for example, JP-B 45-1054, JP-A 52-5709, JP-B 58-46496, and JP-A 61-126050); and a sulfur-based polymerization inhibitor such as a phenothiazine compound or a thiourea-based compound (see, for example, JP-B 59-18378, JP-A 60-89447, and JP-A 8-40979).

In addition, investigation using a naphthoquinone derivative has been known as a method of preventing the polymerization of (meth)acrylic acid and esters thereof (see, for example, JP-A 9-316022).

EP 0 297 583 A describes a photosensitive material comprising at least a polymer precursor, a photosensitive polymerization-controlling agent and a polymerization initiator. Examples of the photosensitive polymerization-controlling agent include inter alia 4-methoxy-1-naphtol, 2-methyl-4-methoxy-1 naphtol, 4-ethoxy-1-naphtol, 2-methoxy-1-naphotl and 4-methoxy-7-methyl-1-naphtol.

US 3,563,742 A relates to photopolymerizable (light-sensitive) compositions that comprise a film-forming oxygen-permeable binder, a polymerizable ethylenically unsaturated monomer, 0.01-5% (by weight of the monomer) of a photolysable naphth - 1 - ol polymerization inhibitor and, optionally, a sensitizing compound which sensitizes the photolysability of the inhibitor.

However, EP 0 297 583 A and US 3,563,742 do not disclose the use of compounds of formula (II) as a polymerization inhibitor for an easily polymerizable compound in a distillation performed in a distillation column, as defined in claim 1 of the present patent, nor do they disclose a method for producing an easily polymerizable compound comprising a distilling step of an easily polymerizable compound in a distillation column, wherein the compound of formula (II) is employed as a polymerization inhibitor, as defined in claim 3 of the present patent.

### DISCLOSURE OF THE INVENTION

An object of the present invention is to provide a novel use of a compound of formula (II) as a polymerization inhibitor that suppresses the polymerization of an easily polymerizable compound, as defined in claim 1.

Another object of the present invention is to provide a novel method capable of suppressing the polymerization of an easily polymerizable compound, as defined in claim 3.

To achieve the above objects, the inventors of the present invention have made extensive studies on a polymerization inhibitor and a polymerization inhibition method for an easily polymerizable compound. As a result, they have found that a naphthol compound typified by an alkoxy naphthol has an excellent polymerization inhibition effect, thereby completing the present invention.

That is, the present invention provides the use of a compound of formula (II) described later as a polymerisation inhibitor for an easily polymerizable compound, as defined in claim 1, and a method employing the compound of formula (II), as defined in claim 3. 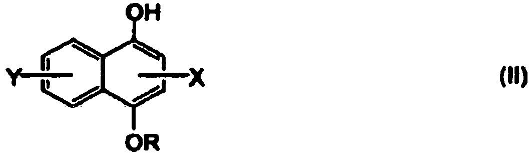 wherein in the formula
X and Y each represent hydrogen, and R represents a methyl group, an ethyl group, an n-propyl group, an i-propyl group, an n-butyl group, or a 2-ethylhexyl group.

Of these, the compounds wherein R is a methyl group, an ethyl group, a n-propyl group or an n-butyl group are particularly preferable.

The compound used as polymerization inhibitor in the present invention is used for preventing the polymerization of an easily polymerizable compound, selected from (meth)acrylic acid and a (meth)acrylate.

Examples of such an easily polymerizable compound include: acrylic acid and methacrylic acid; and (meth)acrylates, such as methyl acrylate, ethyl acrylate, butyl acrylate, 2-ethylhexyl acrylate, octyl acrylate, 2-hydroxyethyl acrylate, 2-hydroxypropyl acrylate, methyl methacrylate, and butyl methacrylate.

The form of the easily polymerizable compound composition in which the compound of formula (II) is used as a polymerization inhibitor and the content of an easily polymerizable compound in the composition are not particularly limited so long as the easily polymerizable compound composition is the composition which comprises the easily polymerizable compound. Examples of such an easily polymerizable compound composition include an easily polymerizable compound itself and a solution of an easily polymerizable compound.

The polymerisation inhibitor composition disclosed herein may contain other component other than the compound used as polymerization inhibitor in the present invention. Such other component is not particularly limited, and examples of the component include compounds conventionally known as polymerization inhibitors.

For example, the polymerisation inhibitor composition may contain, as a nitrogen-containing compound, at least one compound selected from a thioether-based compound, an amine-based compound, and a nitroso compound. Examples of the thioether-based compound include phenothiazine and distearyl thiodipropionate.

Examples of the amine-based compound include p-phenylenediamine, 4-aminodiphenylamine, N,N'-diphenyl-p-phenylenediamine, N-i-propyl-N'-phenyl-p-phenylenediamine, N-(1,3-dimethylbutyl)-N'-phenyl-p-phenylenediamine, N,N'-di-2-naphthyl-p-phenylenediamine, diphenylamine, N-phenyl-β-naphthylamine, 4,4'-dicumyl-diphenylamine, and 4,4'-dioctyl-diphenylamine.

Examples of the nitroso compound include N-nitrosodiphenylamine, N-nitrosophenylnaphthylamine, N-nitrosodinaphthylamine, p-nitrosophenol, nitrosobenzene, p-nitrosodiphenylamine, and α-nitroso-β-naphthol.

Further examples of the nitrogen-containing compound include: piperidine-1-oxyl; pyrrolidine-1-oxyl; 2,2,6,6-tetramethyl-4-oxopiperidine-1-oxyl; and 2,2,6,6-tetramethylpiperidine-1-oxyl.

The polymerization inhibitor composition may also contain at least one kind of compound selected from the group consisting of a phenol compound, a hydroquinone compound, and a quinone compound. Examples of such a compound include: hydroquinone; p-methoxyphenol; cresol; t-butylcatechol; 3,5-di-t-butyl-4-hydroxytoluene; 2,2'-methylenebis(4-methyl-6-t-butylphenol); 2,2'-methylenebis(4-ethyl-6-butylphenol); and 4,4'-thiobis(3-methyl-6-t-butylphenol).

The polymerization inhibitor composition may further contain a copper salt compound and/or a manganese salt compound. Examples of such a compound include: copper salts such as copper dialkyldithiocarbamate (alkyl groups each represent a methyl group, an ethyl group, a propyl group, or a butyl group, and the groups may be identical to or different from each other), copper acetate, copper salicylate, copper thiocyanate, copper nitrate, copper chloride, copper carbonate, copper hydroxide, and copper acrylate; and manganese salts such as manganese dialkyldithiocarbamate (alkyl groups each represent a methyl group, an ethyl group, a propyl group, or a butyl group, and the groups may be identical to or different from each other), manganese diphenyldithiocarbamate, manganese formate, manganese acetate, manganese octanoate, manganese naphthenate, manganese permanganate, and manganese ethylenediamine tetraacetate.

Each of those other components (other polymerization inhibitors) may be used alone, or two or more kinds of them may be used simultaneously. Those other components can be appropriately selected in accordance with the kind, application, and the like of an easily polymerizable compound as an applying object.

The other component to be combined with the compound used as polymerization inhibitor in the present invention is preferably at least one kind selected from hydroquinone, p-methoxyphenol, phenothiazine, copper carbonate, copper dibutyldithiocarbamate, manganese acetate, and the like.

A compounding ratio between the polymerization inhibitor and the other polymerization inhibitor as the other component is arbitrary, but is generally selected from the range of 1 : 100 to 100 : 1, or preferably selected from the range of about 1 : 10 to 10 : 1 on the basis of the polymerization inhibitor. The compounding ratio of the polymerization inhibitor of less than the range may not provide a sufficient polymerization inhibition effect. The ratio in excess of the range provides a sufficient polymerization inhibition effect, but is not economical.

### <Easily polymerizable compound composition>

The easily polymerizable compound composition comprises: the compound used as polymerization inhibitor in the present invention and the easily polymerizable compound. The compounding ingredients of the easily polymerizable compound composition are not particularly limited so long as the composition comprises: the compound used as polymerization inhibitor in the present invention; and the easily polymerizable compound. The composition may contain another component to the extent that an effect of the present invention is not impaired.

A method of adding the compound of formula (II) used as a polymerization inhibitor to the easily polymerizable compound may be the one generally and conventionally known, and is not particularly limited. For example, there may be employed a method involving directly adding the compound of formula (II) in a solid or powder form to (meth) acrylic acid or a (meth)acrylate, or a method involving: dissolving a compound of formula (II) into an appropriate organic solvent; and adding the solution.

The content of the compound of formula (II) in the easily polymerizable compound composition can be appropriately determined in accordance with the kind, application, state, peripheral environment, and the like of the easily polymerizable compound. For example, the compounding content, which varies in various situations such as storage of the easily polymerizable compound and high temperature treatment in distillation at the time of production, is generally selected from the range of 10 to 5,000 ppm by mass, or preferably selected from the range of 50 to 1,000 ppm by mass from the viewpoints of a sufficient polymerization inhibition effect and economy.

In the case of an easily polymerizable compound composition containing a polymerization inhibitor composition and an easily polymerizable compound, the content of the other component (other polymerization inhibitor) can be appropriately determined in accordance with the kind and the like of the other polymerization inhibitor to be used, in addition to the kind, application, state, and peripheral environment of the easily polymerizable compound. For example, when the easily polymerizable compound is (meth)acrylic acid or a (meth)acrylate, as in the present invention, the compounding content of the other polymerization inhibitor is generally selected from the range of 10 to 5,000 ppm by mass, or preferably selected from the range of 50 to 1,000 ppm by mass from the viewpoints of a sufficient polymerization inhibition effect and economy.

### <Method of producing easily polymerizable compound>

The method of producing an easily polymerizable compound of the present invention includes a step of distilling a composition which comprises the easily polymerizable compound in the presence of the compound used as polymerization inhibitor in the present invention in a distillation column, and the easily polymerizable compound is produced from a component obtained in the distilling step. The method of producing an easily polymerizable compound of the present invention is not particularly limited so long as the method includes the distilling step.

In the method of producing an easily polymerizable compound of the present invention, an additional step other than the distilling step may also be performed in the presence of the polymerization inhibitor. Such an additional step is not particularly limited so long as the step is a step in which the easily polymerizable compound is present in a liquid form or a step in which the easily polymerizable compound is heated and becomes to be easy to polymerize.

The component obtained in the distilling step may be a fraction obtained by condensing vapor, or may be a bottoms. In the production method of the present invention, the polymerization inhibitor may be directly, or may be used as the polymerization inhibitor composition, or may be used as the easily polymerizable compound composition.

The distillation column used in the distilling step may be a typical fractionating column in which liquid and vapor can be brought into countercurrent contact with each other. An example of such a fractionating column includes a fractionating column including: a still in which a liquid composition of the easily polymerizable compound is supplied; a reboiler for heating the liquid in the still; a tower such as a perforated plate tower, a bubble cap tower, a plate column, or a packed tower through which vapor generated by the heating passes; a condenser for condensing the vapor distilled from the top of the tower; a reflux pipe for refluxing part of the resultant condensate to the tower; and a decompression device for reducing the pressure in the fractionating column.

The method of producing an easily polymerizable compound of the present invention is a method of producing (meth)acrylic acid or a method of producing a (meth)acrylate.

As the method of producing (meth)acrylic acid, a typical method of producing (meth)acrylic acid may be adopted. An example of such a method of producing (meth) acrylic acid includes a method including the steps of:
(1) producing crude (meth)acrylic acid through a vapor-phase catalytic oxidation reaction of propane, propylene, or isobutylene;
(2) allowing an aqueous medium to absorb the crude (meth) acrylic acid produced in the step (1) to produce an aqueous solution of the crude (meth)acrylic acid;
(3) distilling the aqueous solution of the crude (meth) acrylic acid produced in the step (2) together with a solvent that performs an azeotrope with water and shows liquid-liquid separation to produce a bottom component obtained by removing a low boiling component from the aqueous solution of the crude (meth)acrylic acid;
(4) distilling the bottom component produced in the step (3) to distill off an impurity having a boiling point lower than that of (meth) acrylic acid from the top of the tower; and
(5) fractionating the bottom component produced in the step (4) to obtain (meth) acrylic acid from the top of the tower. The steps (3) and (4) may be performed in one device.

A conventionally known catalyst such as a molybdenum-based composite oxide can be used in the gas phase catalytic oxidation reaction in the step (1). In addition, the aqueous medium in the step (2) is water itself or a liquid medium mainly composed of water; for example, an aqueous medium to be newly supplied, or an aqueous medium which is discharged in another process and the composition or the like of which is adjusted as required may be used.

The term "solvent that performs an azeotrope with water and shows liquid-liquid separation" in the step (3) refers to an organic solvent that is used for liquid separation from water and performs an azeotrope with water. Examples of an available solvent include normal butyl acetate, isobutyl acetate, isopropyl acetate, methyl isobutyl ketone, toluene, heptane, cyclohexane, and diisobutyl ether.

The low boiling component in the step (3) is a component that is distilled off through azeotropy between water and the solvent in the step (3), and examples of the component include water and acetic acid. In addition, examples of the impurity in the step (4) include water and acetic acid.

In the method of producing (meth)acrylic acid, the polymerization inhibitor can be added to a composition of (meth) acrylic acid at an arbitrary point of time before the distillation of the composition of (meth)acrylic acid. For example, in the case of the method of producing (meth) acrylic acid, the polymerization inhibitor is preferably added to the part or whole of the steps (2) to (5) out of the steps (1) to (5).

As the method of producing a (meth)acrylate, a typical method of producing a (meth) acrylate may be adopted. An example of such a method of producing a (meth)acrylate includes a method including the steps of:
(1) subjecting (meth)acrylic acid and an alcohol to an esterification reaction in the presence of a catalyst to produce a crude (meth)acrylate reaction liquid;
(2) recovering the catalyst from the crude (meth)acrylate reaction liquid obtained in the step (1) when the catalyst is an organic acid;
(3) removing unreacted (meth)acrylic acid from the crude (meth) acrylate reaction liquid obtained in the step (1) or the step (2);
(4) distilling the crude (meth)acrylate reaction liquid obtained in the step (3) to distill off an impurity having a boiling point lower than that of a (meth) acrylate from the top of the tower; and
(5) fractionating a bottom component obtained in the step (4) to obtain a (meth)acrylate from the top of the tower.

The alcohol in the step (1) can be appropriately selected in accordance with the kind (structure) of a (meth)acrylate to be produced. In addition, a catalyst generally used for esterification of (meth) acrylic acid can be used as the catalyst in the step (1). Examples of such a catalyst include: inorganic acids such as sulfuric acid and phosphoric acid; organic acids such as methanesulfonic acid, benzenesulfonic acid, and p-toluenesulfonic acid; and strongly acidic cation-exchange resins.

A method with which the catalyst can be recovered can be used without particular limitation for the recovery of the catalyst in the step (2). For example, the recovery can be performed through an operation such as extraction using water.

A method with which (meth) acrylic acid can be removed from the crude (meth) acrylate reaction liquid can be used without particular limitation for the removal of unreacted (meth)acrylic acid in the step (3). For example, the removal can be performed through an operation such as extraction using an alkaline aqueous medium. An example of the impurity in the step (4) includes a raw material alcohol.

In the method of producing a (meth)acrylate, the polymerization inhibitor can be added to a composition of a (meth) acrylate at an arbitrary point of time before the polymerization of the composition of the (meth)acrylate or before the (meth)acrylate is present in a condition under which the (meth)acrylate is of liquid and is polymerizable. For example, in the case of the method of producing a (meth)acrylate, the polymerization inhibitor is preferably added to the part or whole of the steps (1), (4), and (5) out of the steps (1) to (5).

With regard to a method of supplying the polymerization inhibitor in the production method of the present invention, more specifically, the polymerization inhibitor is preferably added to the easily polymerizable compound composition immediately before the distillation of the easily polymerizable compound composition in each of the steps in each of the production methods. Industrially, the polymerization inhibitor can be supplied: by dissolving into a raw material liquid of (meth) acrylic acid or of a (meth) acrylate to be supplied to the fractionating column; or by dissolving into a liquid to be refluxed to the tower or the still by the distillation; or in a liquid state.

The distillation of a composition containing acrylic acid or an acrylate is generally performed under the condition of about 50 to about 150°C and about 13.3 to about 66.7 kPa. According to the present invention, the polymerization of an easily polymerizable compound such as acrylic acid can be sufficiently prevented even in such treatment in a high temperature state, so continuous operation for a long period of time can be achieved.

In each of the methods of producing an easily polymerizable compound composition and an easily polymerizable compound of the present invention, molecular oxygen can be used in combination with the polymerization inhibitor of the present invention. The molecular oxygen may be an oxygen gas itself, or may be an oxygen gas diluted with an inactive gas such as nitrogen. However, a method involving the use of air is economically preferable.

The molecular oxygen may be supplied by being directly mixed with (meth) acrylic acid and a (meth) acrylate through air bubbling or the like. Alternatively, the molecular oxygen may be indirectly supplied to (meth)acrylic acid and a (meth)acrylate dissolved in another solvent. Air bubbling can be easily incorporated into the production process if the molecular oxygen is supplied in a gas state from the bottom of a distillation column or a stripper, or from a reboiler. The molecular oxygen is preferably supplied in an amount of 0.05 to 1 vol% with respect to the distilled vapor amounts of (meth)acrylic acid and a (meth)acrylate.

In the present invention, the polymerization inhibitor is used for an easily polymerizable compound (especially (meth)acrylic acid or a (meth)acrylate), whereby the polymerization of the easily polymerizable compound is effectively prevented. The present invention can be effectively applied to a step of distilling and purifying (meth)acrylic acid or a (meth)acrylate under reduced pressure.

### EXAMPLES

Hereinafter, several specific examples of the present invention will be described, but the present invention is not limited to these.

### Test Example 1. Polymerization prevention test

### <Example 1>

2 g of acrylic acid prepared by recrystallizing commercially available acrylic acid and removing a polymerization inhibitor were placed in a test tube. 2 g of distilled water were added to the test tube to prepare an aqueous solution of acrylic acid. 60 ppm by mass of 4-methoxy-1-naphthol were added to the aqueous solution of acrylic acid. The test tube was capped with septum, and nitrogen was ventilated in the aqueous solution of acrylic acid for 3 min at 15 mL/min. Next, the test tube was immersed in an oil bath heated to 107°C for 10 min. After that, the test tube was taken out from the oil bath, and the viscosity of the aqueous solution of acrylic acid was measured. As a result, the viscosity of the aqueous solution of acrylic acid remained unchanged, so no polymerization was observed.

### <Example 2>

2 g of acrylic acid prepared by recrystallizing commercially available acrylic acid and removing a polymerization inhibitor were placed in a test tube. 2 g of distilled water were added to the test tube to prepare an aqueous solution of acrylic acid. 60 ppm by mass of 4-ethoxy-1-naphthol were added to the aqueous solution of acrylic acid. The test tube was capped with septum, and nitrogen was ventilated in the aqueous solution of acrylic acid for 3 min at 15 mL/min. Next, the test tube was immersed in an oil bath heated to 107°C for 10 min. After that, the test tube was taken out from the oil bath, and the viscosity of the aqueous solution of acrylic acid was measured. As a result, the viscosity of the aqueous solution of acrylic acid remained unchanged, so no polymerization was observed.

### <Example 3>

2 g of acrylic acid prepared by recrystallizing commercially available acrylic acid and removing a polymerization inhibitor were placed in a test tube. 2 g of distilled water were added to the test tube to prepare an aqueous solution of acrylic acid. 60 ppm by mass of 4-butoxy-1-naphthol were added to the aqueous solution of acrylic acid. The test tube was capped with septum, and nitrogen was ventilated in the aqueous solution of acrylic acid for 3 min at 15 mL/min. Next, the test tube was immersed in an oil bath heated to 107°C for 10 min. After that, the test tube was taken out from the oil bath, and the viscosity of the aqueous solution of acrylic acid was measured. As a result, the viscosity of the aqueous solution of acrylic acid remained unchanged, so no polymerization was observed.

### <Example 4>

2 g of acrylic acid prepared by recrystallizing commercially available acrylic acid and removing a polymerization inhibitor were placed in a test tube. 2 g of distilled water were added to the test tube to prepare an aqueous solution of acrylic acid. 60 ppm by mass of 4-methoxy-1-naphthol were added to the aqueous solution of acrylic acid. The test tube was capped with septum, and air was ventilated in the aqueous solution of acrylic acid for 3 min at 15 mL/min. Next, the test tube was immersed in an oil bath heated to 107°C for 10 min while air was blown into the aqueous solution of acrylic acid. After that, the test tube was taken out from the oil bath, and the viscosity of the aqueous solution of acrylic acid was measured. As a result, the viscosity of the aqueous solution of acrylic acid remained unchanged, so no polymerization was observed.

### <Example 5>

2 g of acrylic acid prepared by recrystallizing commercially available acrylic acid and removing a polymerization inhibitor were placed in a test tube. 2 g of distilled water were added to the test tube to prepare an aqueous solution of acrylic acid. 60 ppm by mass of 4-ethoxy-1-naphthol were added to the aqueous solution of acrylic acid. The test tube was capped with septum, and air was ventilated in the aqueous solution of acrylic acid for 3 min at 15 mL/min. Next, the test tube was immersed in an oil bath heated to 107°C for 10 min while air was blown into the aqueous solution of acrylic acid. After that, the test tube was taken out from the oil bath, and the viscosity of the aqueous solution of acrylic acid was measured. As a result, the viscosity of the aqueous solution of acrylic acid remained unchanged, so no polymerization was observed.

### <Example 6>

2 g of acrylic acid prepared by recrystallizing commercially available acrylic acid and removing a polymerization inhibitor were placed in a test tube. 2 g of distilled water were added to the test tube to prepare an aqueous solution of acrylic acid. 60 ppm by mass of 4-butoxy-1-naphthol were added to the aqueous solution of acrylic acid. The test tube was capped with septum, and air was ventilated in the aqueous solution of acrylic acid for 3 min at 15 mL/min. Next, the test tube was immersed in an oil bath heated to 107°C for 10 min while air was blown into the aqueous solution of acrylic acid. After that, the test tube was taken out from the oil bath, and the viscosity of the aqueous solution of acrylic acid was measured. As a result, the viscosity of the aqueous solution of acrylic acid remained unchanged, so no polymerization was observed.

### <Comparative Example 1>

The same operation as that of Example 1 was performed except that 1, 4-naphthoquinone was used as a polymerization inhibitor instead of 4-methoxy-1-naphthol. Acrylic acid in the aqueous solution of acrylic acid in the test tube taken out from the oil bath after 10 min was polymerized.

### <Comparative Example 2>

The same operation as that of Example 4 was performed except that 1, 4-naphthoquinone was used as a polymerization inhibitor instead of 4-methoxy-1-naphthol. Acrylic acid in the aqueous solution of acrylic acid in the test tube taken out from the oil bath after 10 min was polymerized.

### Test Example 2. Distillation test

### <Example 7>

Azeotropic separation of an aqueous solution of acrylic acid was performed by using a distilling device comprising a tower bottom portion, a tower top portion, and a packed tower for connecting them. The tower bottom portion is a 500-mL flask made of glass. The tower top portion is equipped with a distillation pipe. The packed tower is equipped with a raw material supply pipe. And as the packed tower, a packed tower having a height of 100 cm, an inner diameter of 5 cm, and a filling height of a filler of 60 cm was used. A coil pack manufactured by Tokyo Tokushu Kanaami was used as the filler.

Used as a raw material to be supplied through the raw material supply pipe was one obtained by preparing in a model fashion a composition obtained by allowing water to absorb crude acrylic acid produced by a vapor-phase catalytic oxidation reaction of propylene.

That is, 51.5 mass% of acrylic acid, 2.5 mass% of acetic acid, 46.0 mass% of water, 200 ppm by mass of methoxyhydroquinone as a polymerization inhibitor with respect to acrylic acid, and 300 ppm by mass of 4-methoxy-1-naphthol as a polymerization inhibitor with respect to acrylic acid were added to prepare a raw material solution.

The raw material solution was supplied to the distillation column at a flow rate of 270 mL/hr. In addition, toluene was used as an azeotropic agent to perform distillation while a condensate of distilled vapor was circulated in a distillation system in such a manner that the condensate was refluxed from the distillation pipe to the packed tower. Operation conditions included: a tower bottom temperature of 90°C; a tower top temperature of 50°C; and a tower top pressure of 24.0 kPa.

In addition, air was supplied at a flow rate of 300 N-ml/hr ("N-ml" represents a volume (mL) in a normal state) to the tower bottom portion through a capillary pipe placed at the tower bottom portion. The tower bottom portion stored a preparation of 89.7 mass% of acrylic acid, 3.7 mass% of acetic acid, 0.3 mass% of water, and 6.3 mass% of toluene, which had been prepared in advance as a composition close to a stationary state. The time from the start of distillation was measured. It was able to perform distillation stably for 190 min.

### <Example 8>

The same operation as that of Example 7 was performed except that 200 ppm by mass of methoxyhydroquinone, 100 ppm by mass of 4-methoxy-1-naphthol, and 200 ppm by mass of phenothiazine as polymerization inhibitors were added to acrylic acid. The time from the start of distillation was measured. It was able to perform distillation stably for 350 min.

### <Comparative Example 3>

The same operation as that of Example 7 was performed except that 4-methoxy-1-naphthol was not added. As a result, a pressure difference between the tower top and the tower bottom portion of the packed tower occurred at 90 min from the start of distillation, so distillation could not be performed stably. So, the operation of the distilling plant was stopped.

### <Comparative Example 4>

The same operation as that of Example 8 was performed except that 4-methoxy-1-naphthol was not added. As a result, a pressure difference between the tower top and the tower bottom portion of the packed tower occurred at 280 min from the start of distillation, so distillation could not be performed stably. So, the operation of the distilling plant was stopped.

### INDUSTRIAL APPLICABILITY

According to the present invention, by using the polymerization inhibitor for the easily polymerizable compounds, especially (meth)acrylic acid and esters thereof, the polymerization of easily polymerizable compounds can be prevented.

Furthermore, the use of the polymerization inhibitor at the time of distillation in the production process of (meth) acrylic acid and esters thereof is more effective from the viewpoint of suppressing the generation of a polymer in the production process to enable continuous operation of a distillation column and the viewpoint of increasing the yield of a product. In particular, the polymerization inhibitor has an excellent polymerization inhibition effect even in a distillation column having a severe purification condition used for purifying an easily polymerizable compound.

## Claims

1. Use of a compound represented by the following general formula (II) as a polymerization inhibitor for an easily polymerizable compound in a distillation in a distillation column,
wherein the easily polymerizable compound is (meth)acrylic acid or a (meth)acrylate: 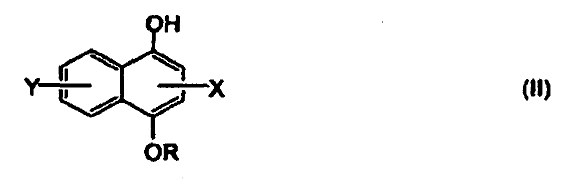 wherein in the formula
X and Y each represent hydrogen, and R represents a methyl group, an ethyl group, an n-propyl group, an i-propyl group, an n-butyl group, or a 2-ethylhexyl group.

2. Use of a compound represented by formula (II) according to claim 1, wherein the easily polymerizable compound is (meth)acrylic acid

3. A method of producing an easily polymerizable compound, which comprises the step of distilling a composition which comprises an easily polymerizable compound in the presence of a polymerization inhibitor in a distillation column;
wherein the polymerization inhibitor is represented by the following formula (II), and the easily polymerizable compound is (meth)acrylic acid or a (meth)acrylate: 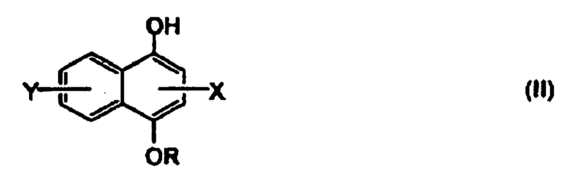 wherein in the formula
X and Y each represent hydrogen, and R represents a methyl group, an ethyl group, an n-propyl group, an i-propyl group, an n-butyl group, or a 2-ethylhexyl group.

4. The method of producing an easily polymerizable compound according to claim 3, wherein the easily polymerizable compound is (meth)acrylic acid.

## Patentansprüche

1. Verwendung einer durch die folgende allgemeine Formel (II) dargestellten Verbindung als Polymerisationsinhibitor für eine leicht polymerisierbare Verbindung in einer Destillation in einer Destillationssäule,
worin die leicht polymerisierbare Verbindung (Meth)acrylsäure oder ein (Meth)acrylat ist: 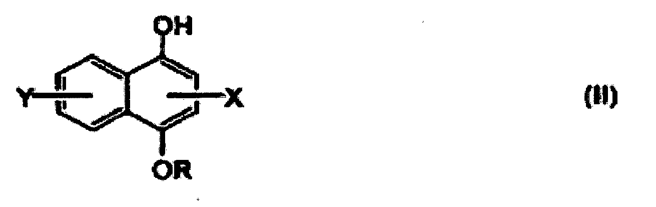
worin in der Formel X und Y jeweils Wasserstoff darstellen und R eine Methylgruppe, eine Ethylgruppe, eine n-Propylgruppe, eine i-Propylgruppe, eine n-Butylgruppe oder eine 2-Ethylhexylgruppe darstellt.

2. Verwendung einer der durch die Formel (II) dargestellten Verbindung gemäß Anspruch 1, worin die leicht polymerisierbare Verbindung (Meth)acrylsäure ist.

3. Verfahren zur Herstellung einer leicht polymerisierbaren Verbindung, welches einen Schritt zum Destillieren einer Zusammensetzung, die eine leicht polymerisierbare Verbindung umfasst, in der Gegenwart eines Polymerisationsinhibitors in einer Destillationssäule umfasst;
worin der Polymerisationsinhibitor durch die folgende Formel (II) dargestellt wird und die leicht polymerisierbare Verbindung (Meth)acrylsäure oder ein (Meth)acrylat ist: 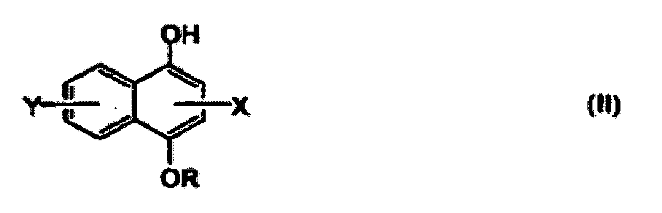
worin in der Formel X und Y jeweils Wasserstoff darstellen und R eine Methylgruppe, eine Ethylgruppe, eine n-Propylgruppe, eine i-Propylgruppe, eine n-Butylgruppe oder eine 2-Ethylhexylgruppe darstellt.

4. Verfahren zur Herstellung einer leicht polymerisierbaren Verbindung gemäß Anspruch 3, worin die leicht polymerisierbare Verbindung (Meth)acrylsäure ist.

## Revendications

1. Utilisation d'un composé représenté par la formule générale suivante (II) en tant qu'inhibiteur de polymérisation pour un composé facilement polymérisable au cours d'une distillation dans une colonne de distillation,
où le composé facilement polymérisable est un acide (méth)acrylique ou un (méth)acrylate : 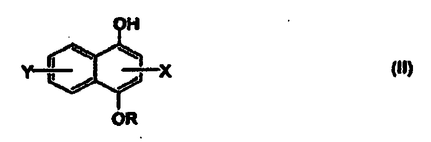 où, dans la formule
X et Y représentent chacun un atome d'hydrogène, et R représente un groupe méthyle, un groupe éthyle, un groupe n-propyle, un groupe i-propyle, un groupe n-butyle, ou un groupe 2-éthylhexyle.

2. Utilisation d'un composé représenté par la formule (II) selon la revendication 1, où le composé facilement polymérisable est un acide (méth)acrylique.

3. Procédé de production d'un composé facilement polymérisable, qui comprend l'étape qui consiste à distiller une composition qui comprend un composé facilement polymérisable en présence d'un inhibiteur de polymérisation dans une colonne de distillation ;
dans lequel l'inhibiteur de polymérisation est représenté par la formule suivante (II), et le composé facilement polymérisable est un acide (méth)acrylique ou un (méth)acrylate : 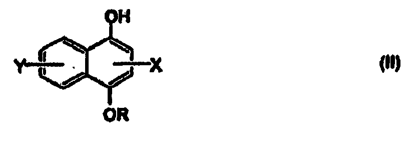 où, dans la formule
X et Y représentent chacun un atome d'hydrogène, et R représente un groupe méthyle, un groupe éthyle, un groupe n-propyle, un groupe i-propyle, un groupe n-butyle, ou un groupe 2-éthylhexyle.

4. Procédé de production d'un composé facilement polymérisable selon la revendication 3, dans lequel le composé facilement polymérisable est un acide (méth)acrylique.
